# FASCICULE DE BREVET EUROPEEN

(11) **EP 0 370 932 B1**
(45) Date de publication et mention de la délivrance du brevet: **28.07.1993**
(21) Numéro de dépôt: 89440118.1
(22) Date de dépôt: 26.10.1989
(51) Int. Cl.: A61F 5/41

(54) **Support pénien**
Penisstütze
Penile support

(30) Priorité: 27.10.1988 FR 8814309
(43) Date de publication de la demande: 30.05.1990
(73) Titulaire: Jost, Didier, F-57000 Metz (FR); Basei, René, F-57070 Metz (FR)
(72) Inventeur: Jost, Didier, F-57000 Metz (FR)
(74) Mandataire: Nuss, Pierre

(56) Documents cités:
- WO-A-84/01284
- WO-A-88/01856
- DE-C- 166 168
- DE-C- 714 925
- DE-C- 875 853
- US-A- 615 337
- US-A- 1 585 861
- US-A- 4 362 152

## Description

La présente invention concerne le domaine médical, en particulier des accessoires de prothèse et/ou de rééducation, notamment pour le traitement de l'impuissance masculine, quelle que soit son origine, médicale, physique et/ou psychique, ou consécutive à un traumatisme avec suite paralysante, et a pour objet un dispositif de prothèse tutrice et/ou de rééducation pour pénis.

La perte des possibilités d'érection, qui se caractérise par la transformation de l'effet de flascidité du pénis en un état rigide par afflux sanguin dans les tissus érectiles, peut être d'origine physiologique accidentelle, momentanée ou définitive ou encore psychologique et rend absolument impossible tout rapport sexuel.

Il existe actuellement certains remèdes classiques pour cet état, qui est gravement infirmant pour un homme et généralement dénommé l'impuissance, à savoir des remèdes médicamenteux, chirurgicaux ou encore suggestifs. Cependant, certains de ces remèdes pris sans surveillance médicale peuvent avoir des effets secondaires graves.

Il a également été proposé de traiter cet état par la psychothérapie qui entraîne un temps de traitement généralement très long avec des chances de succès relativement limitées, voire totalement inefficaces dans le cas de certaines paralysie.

Par ailleurs, on connaît aussi des dispositifs destinés à être greffés à l'intérieur du pénis et pouvant être actionnés mécaniquement. Toutefois, de tels dispositifs nécessitent une intervention chirurgicale délicate et entraînent des frais d'implantation très élevés, sans permettre pour autant d'obtenir toujours des résultats valables et ne peuvent en aucun cas servir à une thérapeutique de rééducation.

La présente invention a pour but de pallier ces inconvénients.

On connait églalement des dispositifs tuteurs, formant uniquement soutien, tels que définis dans le document DE-C-714 925.

Elle a, en effet, pour objet un dispositif de prothèse tutrice et/ou de rééducation pour pénis caractérisé en ce qu'il est essentiellement constitué par un moyen amovible d'élongation et de rigidification du pénis s'appuyant à une extrémité sur le pubis de l'utilisateur et à son autre extrémité sous le gland pour le pousser en avant vers une position extrême.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
la figure 1 est une vue en perspective d'un dispositif conforme à l'invention ;
la figure 2 est une vue partielle en perspective représentant la fixation du moyen d'appui sous le gland ;
la figure 3 est une vue analogue à la figure 2 d'une variante de réalisation de la fixation du moyen d'appui sous le gland ;
la figure 4 est une vue analogue à celle de la figure 1 d'une variante de réalisation du dispositif ;
la figure 5 est une vue en perspective du moyen d'appui sur le pubis du dispositif selon la figure 4, et
la figure 6 est une vue en perspective d'une autre variante de réalisation du dispositif conforme à l'invention.

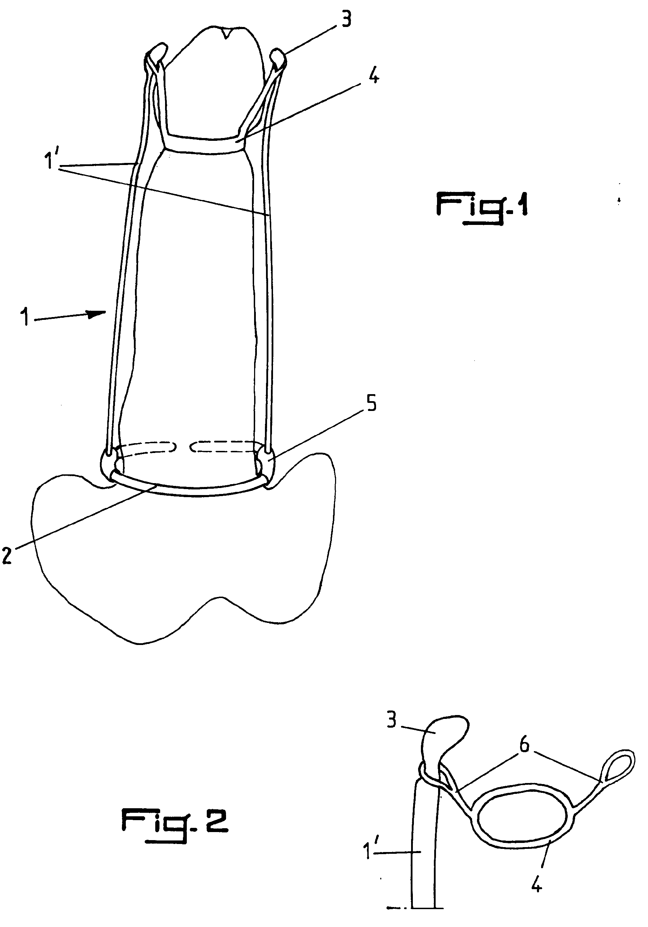

Conformément à l'invention, et comme le montre plus particulièrement la figure 1 des dessins annexés, le dispositif de prothèse tutrice et/ou de rééducation pour pénis est caractérisé en ce qu'il est essentiellement constitué par un moyen amovible 1 d'élongation et de rigidification du pénis qui s'appuie à une extrémité sur le pubis de l'utilisateur et à son autre extrémité sous le gland afin de pousser ce dernier en avant vers une position extrême.

Le moyen amovible 1 d'élongation et de rigidification du pénis se présente avantageusement sous forme d'une paire de potences 1' s'étendant le long et de chaque côté du pénis, reliées à leur partie inférieure, avec possibilité de pivotement, à un dispositif 2 d'appui sur le pubis de l'utilisateur et pourvues à leur extrémité libre d'un bord aplati s'étendant parallèlement au gland, chaque extrémité libre présentant, en outre, une partie 3 d'accrochage d'un moyen 4 de soutien du gland.

Le bord aplati des extrémités libres des potences 1' peut être constitué en une mousse et être légèrement arrondi et épouser la forme ou l'arrondi du gland.

Le dispositif 2 d'appui sur le pubis de l'utilisateur est avantageusement constitué sous forme d'un anneau ouvert ou fermé, à diamètre réglable ou non, relié à une extrémité des potences 1' par une articulation à rotule ou élastiquement déformable 5. Ce dispositif 2 est destiné à former une assise d'appui pour les potences 1' sur le scrotum en entourant, sans serrer, la base du pénis, donc sans occasionner une gêne pour l'utilisateur.

Le moyen 4 de soutien du gland peut être constitué, comme le montre la figure 2 des dessins annexés, par un anneau élastique serrant fermement la base du gland et fixé sur la partie d'accrochage 3 des potences 1' au moyen de liens élastiques 6. Ainsi, après mise en place du moyen 4 sous le gland et en arrière de ce dernier et accrochage à l'extrémité des potences 1' au moyen des liens 6, il est réalisée une traction sur le pénis et donc une élongation de ce dernier, cette élongation résultant, d'une part, de l'appui avec pression du dispositif 2 sur le pubis et, d'autre part, de la traction réalisée sur le pénis par l'intermédiaire du moyen 4 accroché à l'extrémité des potences 1' par les liens 6. La pression sur le pubis a déjà, par elle-même, pour effet de produire sur le pénis rétracté une certaine stimulation entraînant une élongation réflexe.

La figure 3 des dessins annexés représente une variante de réalisation de l'accrochage du moyen 4. Dans ce mode de réalisation, l'anneau élastique formant le moyen 4 est pourvu de deux coquilles élastiques 7 diamétralement opposées, s'emmanchant sur l'extrémité correspondante des potences 1' . Ainsi, l'extrémité libre des potences 1 présente une arête plus souple et plus douce, et donc plus rassurante pour la partenaire.

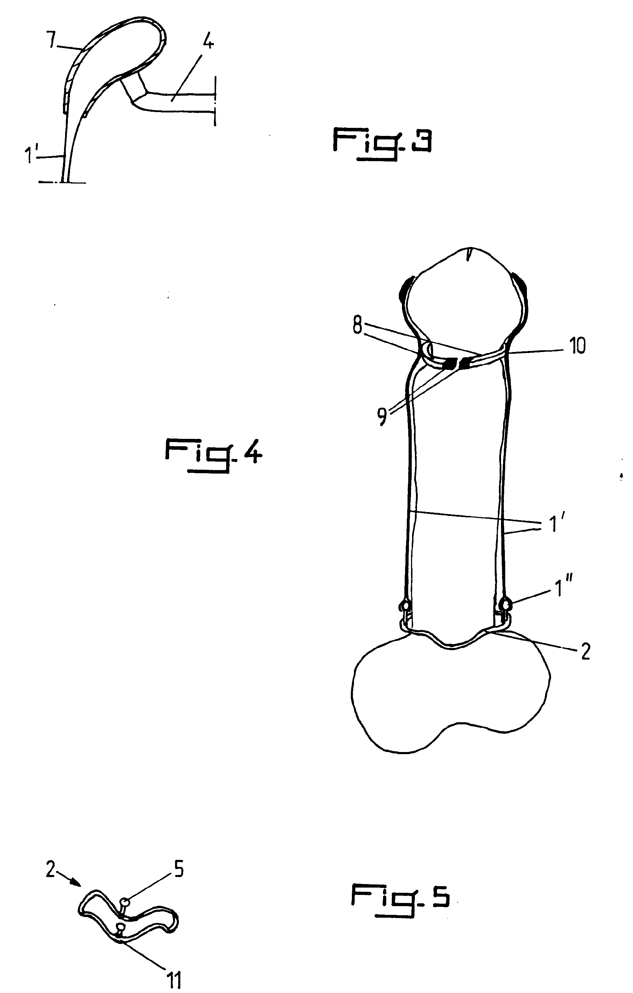

Conformément à une variante de réalisation de l'invention et comme le montre la figure 4 des dessins annexés, les potences 1', montées au moyen de rotules 1'' ou autres articulations sur le dispositif 2 d'appui sur le pubis, sont munies d'un moyen 4 de soutien du gland avantageusement constitué par deux anneaux semi-circulaires 8 présentant à leurs extrémités libres des aimants 9, lesdites potences 1' présentant, au niveau de la fixation des anneaux semi-circulaires 8, une courbure concave 10 correspondant à la zone balono-prépuciale. Par ce mode de réalisation, il est possible de réaliser un dispositif pouvant très facilement être mis en place, par écartement des potences, et donc des anneaux semi-circulaires 8, qui se referment automatiquement sous le gland, après positionnement de celui-ci, sous l'effet de l'attraction des aimants 9. En outre, la courbure 10 est réalisée afin d'éviter une compression du gland ou du pénis lorsque les anneaux semi-circulaires 8 se rejoignent sous l'effet de l'attraction des aimants 9 et que l'anneau ainsi refermé comprime uniquement et suffisamment la zone balono-prépuciale afin de retenir le gland qui reste néanmoins pratiquement entièrement libre.

Le dispositif 2 d'appui sur le pubis (figure 5) présente avantageusement, une courbure concave 11 au niveau des zones d'appui plus déprimables correspondant aux articulations 5 des potences 1'. La courbure 11 permet une légère pénétration de l'anneau formant le dispositif 2 dans les zones de moindre résistance anatomique et entraîne donc une élongation ou un étirement complémentaire du pénis, si souhaité.

Le dispositif conforme à l'invention peut être réalisé sur mesure pour chaque utilisateur. Cependant, il peut également être réalisé de manière adaptable à des utilisateurs d'anatomie différente, par exemple pour un réglage par l'utilisateur lui-même.

Conformément à une caractéristique de l'invention, non représentée aux dessins annexés, les potences 1' sont avantageusement réglables en longueur par réalisation sous forme d'éléments téléscopiques, soit vissés, soit à emmanchement mutuel avec charge par ressort précomprimé ou par aimants répulsifs, soit à emmanchement coulissant avec réglage de position par crans, etc... Un tel mode de réalisation des potences 1' permet une adaptation à la longueur du pénis de l'utilisateur tout en maintenant une certaine traction sur cette dernière, cette traction pouvant varier en fonction des utilisateurs.

Les matériaux pour la réalisation du dispositif conforme à l'invention sont avantageusement des matériaux bio-compatibles tels que les résines et composites utilisés dans les domaines chirurgicaux et dentaires ou les métaux et alliages quaternaires, tels que Cr-Co-Pt, utilisés pour la réalisation de prothèses.

Selon une autre caractéristique de l'invention, non représentée aux dessins annexés, les potences 1' sont avantageusement fixées sur l'articulation 5 du dispositif 2 par un dispositif de liaison démontable telle qu'une liaison à baïonnette ou autre. Une telle liaison permet d'assurer le maintien non démontable des potences sur le dispositif 2, en position d'utilisation, tout en favorisant leur démontage aprés.

Conformément à une autre variante de réalisation de l'invention, non représentée aux dessins annexés, le moyen amovible 1 peut également être constitué par au moins une potence de forme semi-cylindrique s'étendant sous le pénis et reliée, d'une part, à une extrémité, au dispositif 2 d'appui sur le pubis et, d'autre part, à l'autre extrémité, au moyen 4 de soutien du gland, ou par plusieurs potences en forme de portions de cylindre se refermant en position de service à la manière d'un cylindre autour du pénis, entre le pubis et le gland, ces portions pouvant être reliées entre elles par des éléments élastiques.

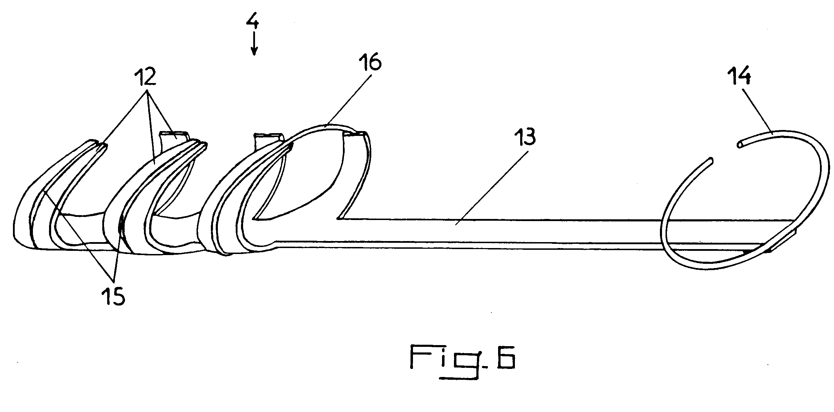

La figure 6 des dessins annexés représente une autre variante de réalisation de l'invention, dans laquelle le moyen 4 de soutien du gland est constitué par plusieurs anneaux semi-circulaires 12 disposés sur une potence 13 s'appuyant sur le pubis au moyen d'un anneau 14, lesdits anneaux semi-circulaires 12 étant situés à un espacement croissant de l'anneau d'appui 14 et étant munis chacun d'une gorge 15 de logement d'un anneau élastique 16 en caoutchouc ou autre, destiné à entourer le gland.

Les anneaux semi-circulaires 12 forment partie intégrante de la potence 13 et sont réalisés en une matière, dont l'âme peut être facilement coupée ou sciée et dont la partie de peau est constituée par une matière molle, telle que de la mousse, la gorge 15 de logement de chaque anneau étant prévue dans cette partie molle. Ainsi, il est possible de régler le dispositif selon la figure 6 à une longueur de pénis donnée, en coupant sim plement les anneaux circulaires en excès en longueur. La prévision d'une partie extérieure molle pour les anneaux semi-circulaires 12 pemet de loger entièrement l'anneau élastique 16 dans lesdits anneaux, de sorte qu'il ne subsiste aucune partie en saillie sur ces anneaux, susceptible de provoquer une irritation.

Le dispositif conforme à l'invention peut également être utilisé en combinaison avec un préservatif et permettra de garantir un maintien absolument fiable de ce dernier par le reserrement possible à la partie extrême du déroulement nécessaire sous le dispositif 2 d'appui sur le pubis qui présente un diamètre légèrement supérieur. Dans ce dernier cas, le dispositif de prothèse, qui est déjà très peu visible sans préservatif, peut d'ailleurs passé totalement inaperçu.

Grâce à l'invention, il est possible de réaliser un dispositif amovible pouvant être utilisé par un homme impuissant et permettant un soutien du pénis, ainsi que procurer une auto-stimulation mécanique et psychique, de mise en place très rapide et permettant un rapport normal en respectant un contact muqueux entre pénis et vagin quasiment total chez les partenaires pendant l'acte sexuel.

En outre, ce dispositif, qui peut servir de prothèse et/ou d'appareil de rééducation, permet de prolonger l'acte, suivant le souhait de l'utilisateur, en cas d'éjaculation précoce, pathologique, qui est en somme une autre forme d'insuffisance.

De plus, le dispositif permet de maintenir sans contrainte, ni douleur, ni gêne, le pénis dans sa position d'érection par une légère traction associée à une pression sur le pubis, qui a pour effet un allongement du pénis, l'application du moyen 4 sous le gland étant prévue suffisante pour empêcher tout risque d'échappement de ce dernier.

Par ailleurs, dans le cas de personnes âgées de sexe masculin présentant un pénis fortement rétracté, le dispositif conforme à l'invention permet, lors d'un alitement permanent, d'éviter un langeage et un changement fréquents du malade, ce dernier pouvant alors uriner normalement dans les dispositifs prévus à cet effet, du fait que son pénis pourra être étiré suffisamment en vue de son introduction dans lesdits dispositifs.

Le dispositif conforme à l'invention permet à un homme impuissant de manière définitive ou provisoire, sans intervention chirurgicale, de retrouver une activité sexuelle acceptable et, par le seul fait de la suppression de la cause psychique liée à la crainte d'une défaillance possible, il peut souvent retrouver la confiance nécessaire à la récupération totale.

En outre, le dispositif peut être mis en place relativement longtemps à l'avance, sans gêne pour l'utilisateur, et lui permet même d'uriner.

Enfin, l'utilisateur du dispositif retrouve pratiquement toutes les possibilités d'une érection physiologiquement normale par la suppléance de l'état érectile obtenu par la prothèse, ainsi que de pénétration et d'éjaculation normales, sans aucune sensation de douleur pour les partenaires en ce qui concerne le contact avec ledit dispositif.

De plus, le dispositif de prothèse présente une action auto-stimulatrice du fait de la pression qu'il effectue sur le pubis par le dispositif d'appui 2 et de l'action de traction sur le gland par le moyen 4, une stimulation complémentaire étant réalisée, en outre, par ledit moyen 4 du fait de la légère compression sous le gland. Ainsi, le dispositif de prothèse étant en place, une érection éventuelle pendant le rapport sexuel, par afflux sanguin, est également rendue possible.

Le dispositif conforme à l'invention permet également à un utilisateur, dont l'impuissance est d'or dre psychique, de retrouver une confiance en ses moyens et donc de contrôler entièrement l'acte, ce qui peut souvent entraîner une guérison rapide et totale, encore confortée par une éventuelle assistance bénéfique apportée par des thérapeutiques complémentaires telles que l'homéopathie, l'organothérapie, la phytothérapie et l'acupuncture, ou tout traitement conventionnel sous surveillance médicale.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention.

## Revendications

1. Dispositif amovible de prothèse tutrice et/ou de rééducation pour pénis caractérisé en ce qu'il est constitué par un moyen (1) d'élongation et de rigidification du pénis qui s'appuis à une extrémité sur le pubis de l'utilisateur et à son autre extrémité sous le gland afin de pousser ce dernier en avant vers une position extrême, ce moyen (1) d'élongation et de rigidification du pénis s'étendant le long du pénis et étant relié à sa partie inférieure, avec possibilité de pivotement, à un dispositif (2) d'appui sur le pubis de l'utilisateur et pourvu à son extrémité libre d'un moyen élastique s'étendant sous le gland, afin de pousser ce dernier en avant vers une position extrême

2. Dispositif, suivant la revendication 1, caractérisé en ce que le moyen s'étendant parallèlement au gland est légèrement arrondi et épouse la forme ou l'arrondi du gland.

3. Dispositif, suivant la revendication 1, caractérisé en ce que le dispositif (2) d'appui sur le pubis de l'utilisateur est avantageusement constitué sous forme d'un anneau relié à une extrémité du moyen (1) par une articulation à rotule ou élastiquement déformable (5).

4. Dispositif, suivant l'une quelconque des revendications 1 et 2, caractérisé en ce que le moyen élastique (4) de soutien du gland est constitué par un anneau élastique serrant fermement la base du gland et fixé sur la partie d'accrochage (3) du moyen (1) par l'intermédiaire de liens élastiques (6).

5. Dispositif, suivant l'une quelconque des revendications 1, 2 et 4, caractérisé en ce que l'anneau élastique formant le moyen (4) est pourvu de deux coquilles élastiques (7) diamétralement opposées, s'emmanchant sur l'extrémité correspondante du moyen s'étendant parallèlement au gland.

6. Dispositif, suivant la revendication 1, caractérisé en ce que le moyen (1) comporte une ou plusieurs potences (1'), montées au moyen de rotules (1'') ou autres articulations sur le dispositif (2) d'appui sur le pubis et munies d'un moyen (4) de soutien du gland avantageusement constitué par deux anneaux semi-circulaires (8) présentant à leurs extrémités libres des aimants (9), lesdites potences (1') présentant, au niveau de la fixation des anneaux semi-circulaires (8), une courbure concave (10) correspondant à la zone balono-prépuciale.

7. Dispositif, suivant l'une quelconque des revendications 1, 3 et 6, caractérisé en ce que le dispositif (2) d'appui sur le pubis présente avantageusement, une courbure concave (11) au niveau des zones d'appui plus déprimables correspondant aux articulations (5) des potences (1').

8. Dispositif, suivant la revendication 6, caractérisé en ce que les potences (1') sont avantageusement fixées sur l'articulation (5) du dispositif (2) par un dispositif de liaison démontable telle qu'une liaison à baïonnette ou autre.

9. Dispositif, suivant la revendication 1, caractérisé en ce que le moyen élastique (4) de soutien du gland est constitué par plusieurs anneaux semi-circulaires (12) disposés sur une potence (13) s'appuyant sur le pubis au moyen d'un anneau (14), lesdits anneaux semi-circulaires (12) étant situés à un espacement croissant de l'anneau d'appui (14) et étant munis chacun d'une gorge (15) de logement d'un anneau élastique (16) en caoutchouc ou autre, destiné à entourer le gland.

10. Dispositif, suivant la revendication 9, caractérisé en ce que les anneaux semi-circulaires (12) forment partie intégrante de la potence (13) et sont réalisés en une matière, dont l'âme peut être facilement coupée ou sciée et dont la partie de peau est constituée par une matière molle, telle que de la mousse, la gorge (15) de logement de chaque anneau étant prévue dans cette partie molle.

## Patentansprüche

1. Abnehmbare Stütz- und/oder Heilprothesenvorrichtung für einen Penis,
dadurch gekennzeichnet, daß sie aus einer Längungs- und Versteifungseinrichtung (1) des Penis besteht, welche sich mit ihrem einen Ende am Pubis des Benutzers und mit ihrem anderen Ende unter der Eichel derart abstützt, daß sie letztere in eine Endstellung nach vorn drückt, wobei diese Längungs- und Versteifungseinrichtung (1) des Penis sich längs des Penis erstreckt und mit ihrem unteren Teil schwenkbar mit einer Einrichtung (2) zur Auflage auf dem Pubis des Benutzers verbunden ist und an ihrem freien Ende mit sich unterhalb der Eichel erstreckenden elastischen Mitteln versehen ist, so daß die Eichel nach vorn in eine Endstellung gedrückt werden kann.

2. Vorrichtung nach Anspruch 1, dadurch gekennzeichnet, daß die sich parallel zur Eichel erstreckende Einrichtung leicht gerundet ist und sich an die form bzw. der Rundung der Eichel anpaßt.

3. Vorrichtung nach Anspruch 1, dadurch gekennzeichnet, daß die Vorrichtung (2) zur Auflage auf dem Pubis des Benutzers vorteilhaft in Form eines Ringes ausgebildet ist, der mittels eines Kugelgelenks oder eines elastisch verformbaren Gelenks (5) mit der Einrichtung (1) verbunden ist.

4. Vorrichtung nach einem beliebigen der Ansprüche 1 und 2, dadurch gekennzeichnet, daß die elastische Stützeinrichtung (4) für die Eichel aus einem elastischen Ring besteht, der die Eichel an ihrer Basis eng umspannt und mittels elastischer Bindungen (6) am Aufhängungsteil (3) der Einrichtung (1) befestigt ist.

5. Vorrichtung nach einem beliebigen der Ansprüche 1, 2 und 4, dadurch gekennzeichnet, daß der die Einrichtung (4) bildende elastische Ring mit zwei diametral gegenüberliegenden elastischen Schalen (7) versehen ist, die auf das entsprechende Ende der sich parallel zur Eichel erstreckenden Einrichtung aufgezogen sind.

6. Vorrichtung nach Anspruch 1, dadurch gekennzeichnet , daß die Einrichtung (1) einen oder mehrere Ständer (1') aufweist, die mittels Kugelgelenken (1'') oder anderer Gelenke an der Vorrichtung (2) zur Auflage auf dem Pubis angebracht sind und mit einer Einrichtung (4) zur Stützung der Eichel versehen sind, welche vorteilhaft aus zwei an ihren freien Enden mit Magneten (9) versehesen halbkreisförmigen Ringen (8) besteht, wobei die besagten Ständer (1') im Bereich der Befestigung der halbkreisförmigen Ringe (8) eine konkave Wölbung (10) aufweisen, die dem Bereich des Vorhautansatzes entspricht.

7. Vorrichtung nach einem beliebigen der Ansprüche 1, 3 und 6, dadurch gekennzeichnet, daß die Vorrichtung (2) zur Auflage auf dem Pubis vorteilhaft eine konkave Wölbung (11) im Bereich der den Gelenken (5) der Ständer (1') entsprechenden, leichter eindrückbaren Auflagezonen aufweist.

8. Vorrichtung nach Anspruch 6, dadurch gekennzeichnet, daß die beiden Ständer (1') am Gelenk (5) der Vorrichtung (2) vorteilhaft mittels einer demontierbaren Verbindungsvorrichtung befestigt sind, z.B. mittels eines Bajonettverschlusses o.ä.

9. Vorrichtung nach Anspruch 1, dadurch gekennzeichnet, daß die elastische Einrichtung (4) zur Stützung der Eichel von mehreren halbkreisförmigen Ringen (11) gebildet wird, die an einem sich auf dem Pubis mittels eines Ringes (14) abstützenden Ständer (13) angebracht sind, wobei die besagten halbkreisförmigen Ringe (11) in zunehmendem Abstand zum Auflagering (14) angeordnet sind und jeweils mit einer Nut (15) zur Aufnahme eines die Eichel umgebenden elastischen Ringes (16) aus Gummi o.ä. versehen sind.

10. Vorrichtung nach Anspruch 9, dadurch gekennzeichnet, daß die halbkreisförmigen Ringe (12) einstükig mit dem Ständer (13) ausgebildet sind und aus einem Material hergestellt sind, dessen kern leicht geschnitten bzw. gesägt werden kann, und dessen Hautteil aus einem weichen Material wie Schaumstoff besteht, wobei die Aufnahmenut (15) jedes Ringes in diesem weichen Teil vorgesehen ist.

## Claims

1. Removable penile prosthesis device for support and/or training, characterised in that it consists of a means (1) for elongation and rigidification of the penis which rests, at one end, on the user's pubic bone and, at its other end, beneath the glans so as to push the glans forward towards an extreme position, this means (1) for elongation and rigidification of the penis extending along the penis and being connected at its lower portion, with the possibility of pivoting, to a device (2) for resting on the user's pubic bone and being provided at its free end with an elastic means extending beneath the glans so as to push the glans forward towards an extreme position.

2. Device according to claim 1, characterised in that the means extending parallel to the glans is slightly rounded and assumes the shape or roundness of the glans.

3. Device according to claim 1, characterised in that the device (2) for resting on the user's pubic bone is advantageously made up in the form of a ring connected to one end of the means (1) by a ball or elastically deformable joint (5).

4. Device according to any one of claims 1 and 2, characterised in that the elastic means (4) for supporting the glans consists of an elastic ring firmly gripping the base of the glans and fixed on the portion (3) for attachment of the means (1) via elastic ties (6).

5. Device according to any one of claims 1, 2 and 4, characterised in that the elastic ring forming the means (4) is provided with two diametrally opposed elastic shells (7) which fit on the corresponding end of the means extending parallel to the glans.

6. Device according to claim 1, characterised in that the means (1) comprises one or more area (1') mounted by means of ball or other joints (1'') on the device (2) for resting on the pubic bone and equipped with a glans-supporting means (4) advantageously consisting of two semi-circular rings (8) having magnets (9) at their free ends, said arms (1') having, in the region of fixing of the semi-circular rings (8), a concave curve (10) corresponding to the balono-preputial zone.

7. Device according to any one of claims 1, 3 and 6, characterised in that the device (2) for resting on the pubic bone advantageously has concave curvature (11) in the region of the most depressable resting zones corresponding to the joints (5) of the arms (1').

8. Device according to claim 6, characterised in that the arms (1') are advantageously fixed to the joint (5) of the device (2) by a detachable connecting device such as a bayonet fitting or the like.

9. Device according to claim 1, characterised in that the elastic glans-supporting means (4) consists of several semi-circular rings (12) arranged on a arm (13) resting on the pubic bone by means of a ring (14), said semi-circular rings (12) being located at an increasing distance from the resting ring (14) and each being equipped with a groove (15) for accommodating an elastic ring (16) of rubber or the like intended to surround the glans.

10. Device according to claim 9, characterised in that the semi-circular rings (12) form an integral part of the arm (13) and are product from a material of which the core may easily be cut or sawn and of which the skin portion consists of a soft material, such as foam, the groove (15) accomodating each ring being provided in this soft portion.
